# EUROPEAN PATENT SPECIFICATION

(11) **EP 1 931 222 B1**
(45) Date of publication and mention of the grant of the patent: **15.02.2012**
(21) Application number: 06840918.4
(22) Date of filing: 04.10.2006
(51) Int. Cl.: A23L 1/29, A61K 31/715, A61K 9/00

(54) **GELLING COMPOSITIONS AND METHODS**
GELIERUNGSZUSAMMENSETZUNGEN UND -VERFAHREN
COMPOSITIONS DE GELIFICATION ET PROCEDES

(30) Priority: 05.10.2005 US 723862 P
(43) Date of publication of application: 18.06.2008
(73) Proprietor: FMC Biopolymer AS, 3002 Drammen (NO)
(72) Inventor: RICHARDSON, Johnathan, Craig, York YO43 3Q4 (GB); DETTMAR, Peter, William, East Yorkshire HU12 0PE (GB); GASEROD, Olav, N-3053 Steinberg (NO); HELGERUD, Trond, N-3400 Lier (NO)
(74) Representative: Dunleavy, Kevin James
(86) International application number: PCT/EP2006/009614
(87) International publication number: WO 2007/039294

(56) References cited:
- EP-A- 1 426 043
- WO-A-2004/110178
- NORTON ET AL: "Fluid gels, mixed fluid gels and satiety" FOOD HYDROCOLLOIDS, ELSEVIER, vol. 20, no. 2-3, March 2006 (2006-03), pages 229-239, XP005090488 ISSN: 0268-005X

## Description

### FIELD OF THE INVENTION

The present invention is directed to methods of inducing satiety, suppressing appetite, treating overweight and obesity, as well as delivery of pharmaceutical or nutraceutical products using gelling compositions, as well as methods for making and using such compositions and products. The invention also relates to a composition comprising a polysaccharide, modified polysaccharide or polysaccharide an alginate or alginate salt capable of ionotropic gelation, a source of multivalent cations capable of solubilization at an acidic pH selected from salts of calcium, iron, strontium, barium, chromium, manganese, nickel, cobalt, molybdenum, copper, aluminum and zinc and a lactone of a carboxylic acid capable of providing a controlled release of protons. Sufficient to solubilize said multivalent cations.

### BACKGROUND OF THE INVENTION

Polysaccharides and their derivatives form a diverse class of versatile materials with countless applications in the pharmaceutical, nutraceutical, food and cosmetic industries. These applications are commonly derived from the properties they impart to solutions upon hydration. Following hydration polysaccharides may be used to viscosify or gel aqueous phases depending on the behaviour of their constituent glycosyl chains. Viscosification of an aqueous phase results from transient entanglement of fluctuating disordered polysaccharide chains, whereas gelation occurs following a more permanent chain-chain interaction. Chain-chain interactions occur both intra and intermolecularly and form junction zones, areas of ordered polymer conformation stabilised by non-covalent interactions such as dipole and ionic interaction, hydrogen bonding and hydrophobic association. A stabilised three-dimensional gel network can be formed if sufficient junction zones develop between polysaccharide chains.

Depending on the type of polysaccharide, chain association and gelation may be initiated through a number of mechanisms. For example, lowering the pH or increasing the ionic strength of certain polysaccharide solutions can decrease inter-chain repulsion and facilitate inter-molecular association. Alternatively, encouraging chain association as opposed to chain-solvent interaction can be achieved through temperature modulation or the addition of a low molecular weight hydrophilic molecule. For certain polysaccharides however, inter-chain interaction and gelation occurs in the presence of multivalent cations.

Sodium alginate, an anionic polysaccharide extracted from the *Phaeophyceae* group of algal plants, is an example of a polysaccharide that gels in the presence of multivalent cations. It is composed of two uronic acids, α-L-guluronic acid and β-D-mannuronic acid grouped into homopolymeric segments of guluronic (G blocks) and mannuronic acid (M blocks) respectively and a block of alternating residues (MG blocks). The conformation of α-L-guluronic acid ensures that glycosyl chains composed of G blocks adopt a buckled, ribbon-like conformation. This buckled chain provides a series of hydrophilic cavities in which a multivalent cation can cooperatively bind. A single cation can simultaneously associate with hydrophilic cavities on adjacent polyguluronic acid chains and in doing so form a three-dimensional crosslinked gel network.

Ionic gelation of sodium alginate has been exploited pharmaceutically to develop systems whose function depends on achieving a gelled state in the stomach. Intra-gastric gelation of sodium alginate may be achieved by co-formulating the polysaccharide with an acid soluble calcium salt. Upon ingestion, gastric acid acts on the alginate:calcium salt formulation to solubilise the calcium salt and liberate calcium ions. The free calcium ions are then available to interact with sodium alginate to form a calcium alginate gel.

U.S. Patent application publication no. 2003/118712 A1 (Navarro Y Koren et al.), U.S. Patent application publication no. 2003/0198726 A1 (Navarro Y Koren et al.), and U.S. Patent application publication no. 2004/258826 A1 (Navarro Y Koren et al.), describe liquid compositions that have a low viscosity at pH 6 and above, but below pH 5 forms a viscous matrix. The liquid compositions comprise at least 0.05% w/w of alginate or pectin, a minimum of 5 mg of calcium per 100mL, an oligosaccharide, water, between I and 20 g of protein per 100 mL, and, optionally, a polyol. These compositions may be used to treat overweight patients for the purpose of inducing a satiety effect.

U.S. Patent application publication no. 2003/134027 A1 (te Hennepe, et al.), and U.S. Patent application publication no. 2004/0228903 A1 (te Hennepe, et al.) describe soft drink replacers. The soft drink replacers are used in methods of treatment and/or prevention of overweight in a monogastric mammal by administration of a liquid composition comprising between 0.01 to 5% w/w alginate and/or pectin and 0.01 to 3%w/w acid soluble calcium salt, optionally containing other ingredients including polyols. Prior to ingestion the beverage has a pH of more than 5 and a viscosity below 50 mPas at a shear rate of 100s⁻¹. At pH 3 and 37°C the viscosity increases to 125% of the aforementioned viscosity. The compositions are said to induce a satiety effect.

U.S. Patent application publication no. 2002/0193344 A1 (Wolf, et al.) relates to a method of blunting the postprandial glycemic response to a meal by feeding an acid controlled induced viscosity fiber system. The fiber system comprises an anionic soluble fiber source, selected from alginate, pectin, low methoxy pectin, carrageenan, xanthan and gellan gum and mixtures thereof, a water-insoluble multivalent cation that is ionized in acid and, optionally, a protein source. A liquid meal replacement product formulated using this fiber system has a low viscosity at neutral pH (50-150cPs) but upon acidification becomes viscous. The product is therefore drinkable at neutral pH but upon ingestion, solubilization of the multivalent cation by gastric fluid, generates an intra-gastric viscosity in excess of 300cPs. This acid induced viscosity fiber system is disclosed as having application in promoting weight loss by producing a satiety effect. U.S. Patent application publication no. 2003/0125301 A1 (Wolf, et al.) describes a method similar to U.S. Patent publication no. 2002/0193344 A1, discussed above, for blunting the postprandial glycemic response. The dual fiber system contains a soluble fiber, a water-insoluble multivalent cation, and a lightly hydrolyzed starch.

Alfred, et al, in W02005/020719 A1 describes an aqueous liquid or spoonable edible composition intended to be used in a weight loss or weight control plan. It comprises from 0.1 to 5% w/w of a biopolymer thickening agent which is not denatured or hydrolyzed between pH 2 and 4, such as alginate, and a source of non-solubilized divalent metal ions in an amount of 2 to 30% w/w based on the weight of the polysaccharide. It is proposed that in gastric conditions the polysaccharide liquid forms a gel, which distends the stomach, and induces a satiety effect. The composition may also include at least 1% w/w of protein as this has been found to strengthen the gel network.

Van Laere, et al., in WO2004/110178 A1 describes a composition that upon reconstitution forms a hot liquid fiber product suitable for the treatment and or prevention of obesity. It comprises 0.1 g to 75 g alginate or low methoxylated pectin, a calcium salt insoluble in a neutral or high pH liquid, and an effervescent system that comprises a base that liberates carbon dioxide and an acidic couple to release carbon dioxide. The liquid at a temperature of at least 35°C has a viscosity below 100 mPas at pH 4, which increases to above 250 mPas at pH 3.

British Patent specification no. 1355985 relates to a slow release tablet containing a water-soluble alginate and an alkaline earth metal salt capable of cross-linking the alginate to form a gelled matrix. More specifically it discloses a tablet formulation, containing sodium alginate and a water insoluble calcium salt, that when exposed to the acid medium of gastric juice releases sufficient calcium ions to gel the alginate. Upon ingestion, release of drug from the tablet is retarded as intra-gastric gelation ensures a gel layer forms on the tablet surface. This gel layer acts as a diffusion barrier to drug efflux and water influx and provides a mechanism for sustained drug release.

U.S. Patent no. 4,140,760 (Withington), issued February 20, 1979, discloses a pharmaceutical composition for use in suppression of gastric reflux containing a low viscosity grade sodium alginate, 0.16 to 2.60 parts by weight of sodium bicarbonate per part by weight of sodium alginate and from 0.10 to 1.04 parts by weight of calcium carbonate per part by weight of sodium alginate. When ingested the sodium alginate formulation forms a carbonated, gelatinous raft in the stomach, which floats on the gastric contents and forms a barrier preventing reflux of gastric fluid into the oesophagus. The mechanism by which the raft is formed is initiated by the interaction between the formulation and gastric acid. Calcium carbonate is solubilized in gastric fluid and the released calcium ions cross-link the alginate to form a gel matrix. In addition, acid solubilisation of calcium carbonate and sodium bicarbonate generates carbon dioxide, which becomes trapped in the gelled matrix and forms a buoyant raft capable of floating on the gastric contents.

U.S. Patent no. 5,286,492 (Dettmar, et al.), issued February 15, 1994, discloses use of triclosan in the treatment of diseases of the gastrointestinal tract by administration of solid unit dosage form comprising 200 to 600 mg of alginic acid and/or a sodium, potassium or magnesium salt thereof; 50 to 250 mg of a sodium or potassium carbonate or bicarbonate salt; I to 100 mg triclosan; and 0 to 100 mg calcium carbonate. The solid unit dosage form may additionally include a pharmaceutically acceptable solid carboxylic acid or an acid salt thereof in sufficient amount to neutralize between one quarter and all of the carbonate and/or bicarbonate of the composition.

WO96/33694 A1 relates to a composition reconstituted in water to form a stable suspension that when ingested forms a gel in the stomach acid medium. The composition contains pectin, a source of calcium ions, such as calcium carbonate, magnesium ions to regulate the kinetics of calcium availability and an effervescent pair. The effervescent pair comprises a mineral or organic acid and a carbonate or bicarbonate alkaline or a sodium or potassium glycine carbonate. Phosphoric, citric and fumaric acids are mentioned as possible acids for use in the effervescent pair. The effervescent pair is included in the formulation to facilitate pectin dispersion and ensure it disperses irrespective of the calcium concentration of the water.

The prior art describes compositions containing a polysaccharide, acid soluble multivalent cation and an acidic component that gel in the stomach. It should be noted however, that in these examples the acid is not added to enhance gelation and instead forms part of an effervescent system where its interaction with a mineral salt of carbonate or bicarbonate to generate gas is considered desirable. In the present invention, however, acid is not added for effervescence, but to augment intra-gastric gelation of the composition independent of endogenous acid secretion. Enhancing the intra-gastric gelation of a polysaccharide:acid soluble multivalent cation composition via co-formulation with an acidic component is therefore novel and offers significant advantages compared to compositions described in the prior art.

It is believed that utilizing endogenous acid as the 'trigger mechanism' for gelation is unreliable as the acidity of gastric fluid can be variable. A number of factors may reduce gastric acidity. These include (i) drug therapy, in particular proton pump inhibitors and other drugs that modulate gastric acid secretion; (ii) disease, for example gastritis or *Helicobacter pylori* infection; (iii) diurnal variation resulting from the circadian rhythm; (iv) surgery, for example cholecystectomy; (v) diet; (vi) age and (vii) fed/fasted state. The performance of pharmaceutical compositions whose function relies on intra-gastric gelation is therefore considered to be impaired when administered to individuals with reduced gastric acidity.

Other gelling compositions used for different purposes are also known in the art. For example, WO 2003/090718 discloses an air treatment gel formed from an air treating substance, an alginate or a pectic substance, at least one added polymer which does not gel and less than 100% of a stoichiometric amount of a gel-forming cation such as a calcium cation. A pH modifier that slowly lowers the pH, such as acids that provide a buffering action and/or materials that slowly generate acid, may be included in the composition. Exemplary acids include lactic acid lactone, glycolic acid lactone and glucono delta lactone.

U.S. Patent application publication no. 2005/0137272 discloses a foam gel preparation including an alginate, less than 100% of a stoichiometric amount of a gel-forming cation such as a calcium cation, and a predominant amount of a plasticizer. A pH modifier that slowly lowers the pH, such as acids that provide a buffering action and/or materials that slowly generate acid, may be included in the composition. Exemplary acids include lactic acid lactone, glycolic acid lactone and glucono delta lactone. The time for gelling the foam may be controlled by varying the size of the particles of the gelling agent. Foaming may be induced by mechanical agitation or introduction of a gas, such as air, into the composition.

WO 2005/032273 discloses a fast forming gel including alginate, sugar, water, a calcium salt, an acid and a sequestering agent. The acid may be adipic acid, glucono delta lactone or citric acid.

EP 1426043 is directed to pharmaceutical suspensions containing poorly soluble or insoluble actives, a hydrocolloid and effervescent agents for the purpose of overcoming sedimentation problems by ensuring complete dispersion of both the active compounds and the hydrocolloids.

### SUMMARY OF THE INVENTION

In a first aspect, the present invention relates to cosmetic treatment methods for inducing satiety, sustained release, gastric retention, inhibition of nutrient uptake, anti-reflux therapies, and pharmaceutical and nutraceutical delivery. In the method, a composition comprising (a) analginate or alginate salt capable of ionotropic gelation, (b) at least one source of multivalent cations capable of solubilization at an acidic pH selected from salts of calcium, iron, strontium, barium, chromium, manganese, nickel, cobalt, molybdenum, copper, aluminum and zinc, and (c) at least one lactone of a carboxylic acid capable of providing a controlled release of protons sufficient to solubilize the multivalent cations, is administered to a mammal for the purpose of forming a gel in the stomach of the mammal when ingested, which gel resists peristaltic forces and remains in the stomach for an extended time.

In a second aspect, the present invention relates to a composition that augments the acidity of gastric fluid such that intra-gastric gelation of the polysaccharide:acid soluble multivalent cation formulation is initiated independent of endogenous acid secretion. In this aspect, the present invention embodies a composition as claimed in claim 31.

The present composition provides a means of enhancing the functionality of polysaccharide:acid soluble multivalent cation compositions that rely on intra-gastric gelation for their efficacy. Thus, the composition addresses the potential problem that the stomach pH may be sufficiently high as to inhibit gel formation via co-formulation with an acidic component. It therefore has utility across a broad range of applications including inducing satiety, sustained release, gastric retention, inhibition of nutrient uptake, anti-reflux therapies, and pharmaceutical and nutraceutical applications.

In another aspect, the present invention relates to a variety of edible products and packaged edible products which comprise a composition as claimed in claim 32. (a) at least one of a polysaccharide, modified polysaccharide, or polysaccharide salt, each capable of ionotropic gelation, (b) at least one source of multivalent cations capable of solubilization at an acidic pH, and (c) at least one acid component capable of providing a controlled release of protons sufficient to solubilize the multivalent cations. These edible products are capable of forming a gel in the stomach when ingested that resists peristaltic forces and remains in the stomach for an extended time.

### BRIEF DESCRIPTION OF THE DRAWINGS

Figure 1 is a graph of the relative hunger, post-meal for an example in accordance with the present invention
Figure 2 is a graph of the time until the next eating episode for an example in accordance with the present invention.
Figure 3 is a schematic representation of an apparatus for obtaining an indication of gel strength used in Example 2 of the present application.
Figure 4 is a graph of the force measured using the apparatus of Figure 3 for the Powder Formulation.
Figure 5 is a graph of the forces measured using the apparatus of Figure 3 comparing the Liquid and Powder Formulations.
Figure 6 is a graph of the force measured using the apparatus of Figure 3 for compositions of Example 2 employing different concentrations of alginates.

### DETAILED DESCRIPTION OF THE INVENTION

This invention provides a means of enhancing the functionality of polysaccharide:acid soluble multivalent cation compositions that rely on intra-gastric gelation for their efficacy. The composition used in the methods of the present invention is capable of intra-gastric gelation. However gelation is not reliant on physiological gastric acid secretion and instead is achieved through the choice of a suitable component described herein. Through judicious choice of the acidic component the kinetics of gelation can be controlled. The donation of protons, and therefore gelation kinetics, can be modulated using acids of varying pKa and solubility. Alternatively using lactones of carboxylic acids, which donate protons upon hydrolysis, provides a further method of controlling gelation.

The methods of the invention can be used to treat or prevent obesity in therapy and/or for the cosmetic treatment or prevention of obesity, i.e. losing weight in mammals, and more preferably, in humans. The present method can suitably be used to induce a satiety effect. As a result, the invention also includes a method for appetite reduction. The present method is preferably used to prevent or treat obesity or suppress appetite in mammals including humans by inducing a satiety effect.

One method in accordance with the invention comprises administering an effective amount of a composition comprising (a) analginate or alginate salt capable of ionotropic gelation, (b) at least one source of multivalent cations capable of solubilization at an acidic pH selected from salts of calcium, iron, strontium, barium, chromium, managanese, nickel, cobalt, molybdenum, copper, aluminium and zinc, and (c) at least one lactone of a carboxylic acid capable of providing a controlled release of protons sufficient to solubilize the multivalent cations.

The present method may involve administration of a composition in accordance with the present invention including one or more of the additional and/or optional ingredients of the composition, as described below. The composition may be administered one to five times per day, as needed, to suppress appetite, induce a satiety effect, reduce caloric or food intake and/or treat or prevent obesity.

Furthermore, the present composition may be employed reduces caloric intake of a meal when consumed shortly before or during the same meal. Hence the present invention also provides a method for reducing the caloric intake of a meal, for prevention of ingesting excess calories when ingesting a meal and/or controlling daily caloric intake, said method comprising administering a composition as set forth above shortly before or during a meal.

The present invention also relates to method for controlled drug release by including a pharmaceutical active in any of the compositions described above, resulting in the active being included in a hydrophilic matrix upon gel formation in the stomach, and a subsequent sustained release of the active and/or which after hydration and gelling is retained in the stomach for a certain period of time because it becomes a gel too bulky to pass through the pyloric sphincter.

The present invention also relates to a method for prevention of oesophageal reflux and/or the symptoms of oesophageal reflux by ingesting any of the compositions as described above.

Each of the methods of the invention relies on gelation of the ingested composition in the stomach of a mammal to induce a satiety effect and/or provide a matrix to control delivery of a pharmaceutical or nutraceutical. Also, in the methods of the present invention, control of the time and/or rate of gelation may play an important role. For example, substantial gelation must be prevented from occurring prior to ingestion of the edible product by the mammal. The methods of the present invention address this issue by providing a controlled gelation of the product which permits sufficient time to ingest the product.

The composition and methods of the present invention are capable of intra-gastric gelation, however, gelation is not reliant on gastric acid in the stomach and instead is achieved through the choice of a suitable component that provides release of protons sufficient to solubilize multivalent cation. Suitable compositions are capable of hydrating in aqueous media and subsequently forming a gel in the stomach when ingested, that resists peristaltic forces and remains in the stomach for an extended time. The methods of the invention allow for variations in the pH of the stomach contents of the mammal ingesting the product by employing a composition capable of gelation in the stomach, independent of the pH of the stomach contents. This provides a more reliable induction of a satiety effect and/or delivery of a pharmaceutical or nutraceutical by elimination of a process variable caused by stomach pH, since the methods of the present invention are capable of operation independent of stomach pH. Suitable compositions for use in the methods are described in detail below.

### Compositions for Use in the Methods

In one aspect, the present invention relates to a composition comprising: (a) an alginate or alginate salt each capable of ionotropic gelation, (b) at least one source of multivalent cations capable of solubilization at an acidic pH selected from salts of calcium, iron, strontium, barium, chromium, maganese, nickel, cobalt, molybdenum, copper, aluminum and zinc, (c) at least one lactone of a carboxylic acid capable of providing a controlled release of protons sufficient to solubilize said multivalent cations, wherein the composition is capable of hydrating in aqueous media and subsequently forming a gel in a stomach when ingested that resists peristaltic forces and remains in the stomach for an extended time. The composition may also contain a gas-forming component to increase the volume of the resulting gel, a dispersing agent to facilitate dispersion and dissolution of the full formulation in the stomach, and flavouring and sweetening agents.

The present invention also includes a variety of edible products and packaged edible products which comprise the ingredients of the composition of the present invention. These products are capable of forming a gel in the stomach when ingested that resists peristaltic forces and remains in the stomach for an extended time.

The edible and packaged edible products typically comprise a composition as set forth above.

### Polysaccharide or Derivative Capable of Ionotropic Gelation

A polysaccharide that is used in the present invention is alginate. Alginate is an anionic polysaccharide extracted from the *Phaeophyceae* group of algal plants (brown algae). Alginate comprises up to 40% of the algal plant dry matter and is believed to give strength and flexibility to the algal tissue. In addition to the algal source, alginate may also be synthesised by certain microbial species such as *Aztobacter vinelandii* and some *Pseudomonas* species. Alginate is composed of two uronic acids, α-L-guluronic acid and β-D-mannuronic acid joined by 1,4-glycosidic bonds. The uronic acids are organised into homopolymeric segments of guluronic (G-blocks), mannuronic acid (M-blocks) respectively and a block of alternating acid residues (MG-blocks). The exact uronic acid composition of alginate varies according to the species of brown algae, the age of the algal plant, the season, environmental conditions, and the specific part of the plant sampled.

The uronic acid composition also influences the gel-forming properties of alginate. The strength of the gel formed is dependent on the amount of guluronic acid present and the length of the polyguluronic acid chain. In the preferred composition the alginate should have an L-guluronic acid content of at least 35% of the total uronic acid units, preferably at least 50%, more preferably 60% and most preferably at least 65%. It is preferred that the alginate has a molecular weight average between 0.1 x 10⁵ and 5.0 x 10⁵, preferably 0.2 x 10⁵ to 4.5 x 10⁵, more preferably between 0.3 x 10⁵ to 3.0 x 10⁵ and most preferably between 0.4 x 10⁵ to 2.0 x 10⁵. Suitable alginate salts are water soluble, including monovalent ions and magnesium, preferably monovalent, and in particular sodium, potassium and ammonium. Appropriate alginates according to this embodiment include the Protanal^{®} range available commercially through FMC BioPolymer.

Pectin may also be used in the present invention in addition to alginate. Pectin is a heterogeneous complex polysaccharide found in all higher plants, however commercially it is usually derived from citrus peel or apple pomace. Structurally, pectin is composed of linear segments of 1,4-linked α-D galactopyranosyluronic acid units in which some of the carboxyl groups are esterified with methanol. In addition, the hydroxyl groups of certain sources of pectin are also esterified with acetic acid. Pectin may also be treated with ammonia to form amidated pectin. Pectins with a degree of methoxylation below 50% are termed low methoxylated pectins and are capable of ionotropic gelation. The pectins used in the present invention are characterised by a degree of methoxylation below 50%, more preferably below 40% and most preferably below 35%. Additionally the pectins are amidated, the degree of amidation being between 10-30%, preferably 10-25% and more preferably 10-20%.

The concentration of polysaccharide, modified polysaccharide or polysaccharide capable of ionotropic gelation will depend on the physical form of ingestion. If ingested in solid form the concentration of polysaccharide should be below 90%, preferably below 60% and most preferably below 50% of the total weight of solids. If ingested in liquid form the concentration of polysaccharide should desirably be limited because at high concentrations it can form a solution with an undesirably high viscosity. The concentration of polysaccharide in liquid formulations should desirably be in the range 0.05-20%, preferably 0.1-15% and most preferably 0.5-10% based on the total weight of the liquid composition. When consumed, a preferred dose of the polysaccharide of the of the alginate will be about 0.1-10.0 grams per meal, more preferably 0.5-5.0 grams per meal, and most preferably, 0.5-5.0 grams per meal. This size dose provides the consumer with a reasonable sized portion of the edible product that can be ingested in a short period of time, while at the same time providing a sufficient satiety effect to be a benefit to the consumer.

Carrageenans may also be used in the present invention in addition to alginate. Carrageenans are derived from red seaweed, and are a group of sulphated polysaccharides with a complex structure. This group of high molecular weight polysaccharides consists of repeating galactose units and 3,6-anhydrogalactose, both sulphated and non-sulphated. The units are joined by alternating alpha-1,3 and beta-1,4 glycosidic linkages. Preferred carrageenans are capable of forming ionotropic gels with multivalent ions, like iota-carrageenan. Carrageenans may be used in combination with alginate or pectin or other polysaccharides or modified polysaccharides or polysaccharide salts to modify gel properties, particularly acid stability.

All particle sizes of the polysaccharide or derivate can be used, but the particle size may affect both the dispersion and hydration time of the formulation. To provide a preferred dispersible product that easily dissolves sieved fractions or granulates of the formulation may be used. Sieving provides good flow, which enables the powder mix to be easily dissolved without lumping. Granulation of the whole powder mix provides even better flow characteristics than sieving and may also help to prevent segregation of the product.

### Source of Multivalent Cations Solubilised at an Acidic pH

The composition of the invention contains a source of multivalent cations capable of solubilization at an acidic pH. In the present composition, a reduction in pH occurs when ingested and this is achieved, at least in part, through the co-formulation of the composition with a component capable of providing controlled release of protons sufficient to solubilize the multivalent cations. Multivalent cations that become available in solution following a reduction in pH include (i) multivalent cation salts whose aqueous solubility is increased upon acidification; (ii) a calcium salt coated or complexed with a substance to limit its water solubility that becomes more soluble upon acidification and (iii) mixtures thereof. Numerous multivalent cation salts whose aqueous solubility is increased upon acidification are commercially available and include salts of calcium, iron, strontium, barium, chromium, manganese, cobalt, nickel, molybdenum, copper, aluminium and zinc. In particular salts of calcium are preferred including calcium carbonate, calcium citrate, calcium fluoride, calcium glycerophosphate, calcium hydroxide, calcium oxalate, calcium phosphate dibasic, calcium phosphate monobasic, calcium phosphate tribasic, calcium pyrophosphate, calcium saccharate, calcium succinate, calcium sulfite and calcium tartrate and mixtures thereof.

Further examples of technologies employed to ensure the multivalent ion is released upon acidification are complexation with an acid cleavable moiety and coating in an acid-labile or thermo-labile coat. The concentration of multivalent cation source used in the composition should be sufficient that upon acidification adequate free multivalent cations are available to cross-link the polysaccharide and form a gel. In the preferred composition the source of multivalent cation is calcium. The concentration of calcium that is typically used in the composition should be sufficient that upon acidification the concentration of free multivalent cations will be at least 1% of the polysaccharide weight, more preferably 2.5 - 20% of the polysaccharide weight, and most preferably 5 - 15% of the polysaccharide weight. In one embodiment, the composition comprises at least 100% of a stoichiometric amount of multivalent cations required for reaction with the polysaccharide.

The particle size of the multivalent cation powder may affect the speed of gelation. In particular, a finer particle size will typically increase the speed of gelation. There is, in principle, no limitation to the particle size distribution of the multivalent cation source, but the particle size distribution should be preferably be within 1 - 100 µm in diameter, more preferably 20 - 80 µm in diameter and most preferably 25 - 50 µm in diameter. The multivalent cation may also be used in a granulated form where it has been granulated with a binder system such as, for example, maltodextrin and/or acacia amongst others.

### Component Capable of Providing Controlled Release of Protons Sufficient to Solubilize the Multivalent Cations

This component includes a lactone of a carboxylic acid that provides a controlled release of protons sufficient to solubilise the multivalent cation.

As used herein, "controlled release" of protons means that the composition is engineered so as to provide protons sufficient to solubilize the multivalent cations at a time and/or rate to provide gelation at a particular time or location, or at a particular rate. Depending on the mechanism of delivery, the release of protons can be immediately upon ingestion of the composition. Alternatively, the release of protons can be delayed and/or sustained over a period of time. For example, if the composition is placed in aqueous media and then ingested (e.g., a drinkable composition), the release of protons may be delayed or sufficiently slow that the composition does not gel for a certain period of time to allow a period of time for ingestion of the composition prior to the occurrence of significant gelation. In another example, the composition is engineered such that release of protons is triggered by hydration and/or dissolution of the composition in the oral cavity and/or stomach, and proton release is delayed and/or sustained over an extended time period to facilitate ingestion of the composition, prior to gelation, as well as to facilitate the formation of a firm gel in the stomach.

The composition is capable of forming a defined, firm gel in the stomach, with sufficiently high gel strength to resist peristaltic forces from emptying it from the stomach through the pyloric sphincter for an extended time when the composition is being used, for example, as a satiety product. For example, the gel is capable of staying in the stomach for a sufficient amount of time to induce a feeling of satiety by the consumer.

It is preferred that the acidification of the composition, and therefore the liberation of the multivalent cation and gelation, can be controlled. Control over proton donation may be achieved using lactones of carboxylic acids. Being able to control acidification and therefore gelation is particularly advantageous for reconstitutable compositions. Upon reconstitution, the acidification of the liquid product progresses in a controlled manner such that release of the multivalent cation is delayed and/or sustained over a period oftime. This retards gelation of the product and allows it to remain as a low viscosity liquid for a sufficient period of time to be ingested. Following ingestion, continued acidification of the product by the acidic component ensures that sufficient multivalent cation is released to form a gelatinous mass in the stomach even if the stomach pH is sufficiently high as to inhibit gel formation via co-formulatipn with an acidic component. The present composition thereby provides a means of enhancing the functionality of polysaccharide:acid soluble multivalent cation compositions that rely on intra-gastric gelation for their efficacy. The preferred concentration of the acidic component is dictated by the desired kinetics of gelation and can be suitably determined by those skilled in the art.

The acidic component that provides controlled release of protons should preferably be maintained in a dry condition until just prior to or during ingestion. Thus, it is preferable that, for example, no water be added to the acidic component of the composition, when packaged or stored. Also, it is preferred that the acidic component be packaged in a manner which minimizes contact with atmospheric moisture and/or prevents hydration of the acid component with moisture contained in one or more other components of the composition.

As an example, the acidic component should allow from 1-60 minutes for the composition to under substantial gelation in order to provide the consumer with sufficient time to ingest the material and ensure that gelation occurs in the stomach. Values in the lower end of the range, e.g. 1-10 minutes, are used, for example, in the case of solid compositions that are hydrolyzed in the oral cavity and/or stomach since a shorter time period for ingestion will be required in this case. Beverages and other edible products that must be mixed by the consumer should allow 5-60 minutes for the composition to undergo substantial gelation in order to prevent premature gelation prior to ingestion of the product. More preferably, 5-40 minutes for the composition to undergo substantial gelation is selected, and, most preferably, about 10-30 minutes is selected.

It is desirable not to exceed about 60 minutes since many of the desired effects of the invention will provide more benefit if gelation time is shorter. For example, if it takes too long to induce a satiety effect, the consumer may eat something else before feeling sated, thereby at least partially defeating the purpose of the invention in such case.

The mole ratio of the number of moles of the polysaccharide or derivate to the number of moles of the multivalent cation is preferably less than 30:1, more preferably, less than 22:1, and most preferably, less than 6:1. The mole ratio of the number of moles of acid moieties of the component capable of providing slow release of a proton to the number of moles of the multivalent cation is preferably more than 1:1, more preferably, more than 10:1 and, most preferably, more than 100:1. This mole ratio is chosen to ensure the formation of a firm gel in the stomach by providing a relatively high degree of cross-linking of the polysaccharide component.

### Gas forming component

If desired, a gas forming component may be used. The gas-forming component may include any compound or material that upon contact with acid will release gas. Suitable gas-forming components include compounds releasing carbon dioxide (CO₂) or other non-toxic gases on contact with neutral or acidic aqueous media. This includes bicarbonate salts, in particular alkaline metal bicarbonates like sodium bicarbonate, potassium bicarbonate, lithium bicarbonate, and ammonium bicarbonate. The gas-forming agent should preferably be combined with an acid capable of hydrolysing the bicarbonate relatively quickly, in order to create gas trapped in the gelling liquid and increase the gel volume before the structure is completely gelled. The acid could be chosen from a group of organic and inorganic acids, preferably malic acid, tartaric acid, citric acid, fumaric acid, adipic acid, succinic acid, lactic acid, glycolic acid, alpha hydroxyl acids, and ascorbic acid. Preferably, not more than about 0.5wt% of this organic or inorganic acid is employed in the composition so as to minimize potential interference by this acid component with the activity of the component that provides controlled release of protons, discussed above. Also, preferably the number of moles of protons released by this acid component should be not more than 7x10⁻³ moles of H⁺ per 100 grams of the edible composition.

### Dispersing Agent

If desired, a dispersing agent may be used. The purpose of the dispersing agent is to facilitate rapid dispersion and/or dissolution of the powder formulation in water. The dispersing agent can be solid or liquid, water soluble or insoluble, and is preferably a crystalline material that is soluble in water. The dispersing agent may be a liquid that assists in dispersion through agglomerating one or more of the solid ingredients and modifying hydration properties. A suitable group of dispersing agents may include compounds that consist of crystalline or amorphous particles with a particle size distribution allowing rapid dispersion in aqueous media. Numerous substances are available, like sucrose, fructose, maltose, maltitol, polydextrose, xylitol, sorbitol, mannitol, erythritol, Isomalt™, Sucralose, Stevia derivates, any type of polyol, proteins, or mixtures thereof. Suitable liquids include, but are not restricted to, edible oils, glycerol, polyethylene glycol and other polyols. To the extent that these optional dispersing agents may also function as plasticizers, it is preferred that the ratio of plasticizer to gel forming agent is less than 2:1, and, more preferably, less than 1:1. The optional plasticizer preferably comprises less than 50wt% of the gelled material.

### Flavouring, Colorants and Sweetening Components

If desired, flavouring, colorants and/or sweetening agents may be employed. The powder formulation may contain flavours and sweeteners to increase the palatability of the product. The formulation may at least include one or more flavouring and/or sweetening components.

Flavours can be selected from, but are not restricted to, fruit, berry and chocolate flavours, preferably flavours compatible with a neutral pH value. Preferred flavours are selected from the group of vanilla, banana, strawberry and chocolate.

Sweetening agents can be added if not sufficient sweetening effect is obtained through effects of the dispersing agent. Suitable sweeteners may be selected from the groups of natural and artificial sweeteners, preferably with a low caloric content. Numerous substances are available which may be used for this purpose including, but not limited to, sucrose, fructose, maltose, maltitol, polydextrose, xylitol, sorbitol, mannitol, erythritol, Isomalt™, Sucralose, Stevia derivates, any type of polyol, proteins, or mixtures thereof. Suitable liquids include, but are not restricted to, edible oils, glycerol, polyethylene glycol and other polyols.

### Other Ingredients

The powder formulation can optionally include other ingredients than the above mentioned. This group of components intended for uptake in the gastro-intestinal tract, fillers, or ingredients modifying the kinetics of degrading the gel. This will include, but not be limited to, pharmaceutical actives, nutrients, nutraceuticals, vitamins, minerals, and proteins, probiotics, prebiotics, or other components which are desirably delivered in a controlled way.

Surprisingly, it has been found that the present composition gels to form a matrix with considerably more strength than a composition not containing the component of the present invention capable of providing a controlled release of protons. It is therefore apparent that the present invention provides a means of enhancing or ensuring intra-gastric gelation independent of gastric acidity. Through choice of the component capable of providing release of protons and the use of an amount sufficient to solubilize the multivalent cation, the kinetics of gelation can be controlled. The donation of protons, and therefore gelation kinetics, can be modulated using acids of varying pKa and solubility. Alternatively using lactones of carboxylic acids, which donate protons upon hydrolysis, provides another method of controlling gelation.

Advantageously, the liquid product, prior to gelation, has a viscosity below 100 mPas, more preferably, below 50 mPas, and most preferably, below 25 mPas at a shear rate of 100s⁻¹. Once substantial gelation has occurred, the viscosity of the gelled product may exceed 200 mPas, 500 mPas or even 1000 mPas at a shear rate of 100s⁻¹.

### Edible Forms of the Invention

As a result of the ability to control the gelation time and/or duration of gelation, the invention may be implemented in a variety of different edible forms. In one embodiment, the invention is implemented as a dry powder that may be reconstituted in water. In this embodiment, all of the components of the composition can be packaged together in dry form. Alternatively, one or more of the dry components can be packaged separately from the others and the dry components can be mixed together by the consumer just prior to ingestion. For example, if a beverage were to be provided where the consumer could select from two or more colors and/or flavors, for example, by selection from two or more powder ingredients that impart color and/or flavor, one of the components of the present composition could be included in the flavor and/or color-containing component to allow mixing of the components just prior to ingestion in this manner.

In another form, the invention may be implemented as a multi-component product, wherein one component of the product is a liquid and a second component of the product is a liquid or solid material. In such case, the two or more components of the product may be mixed together by the consumer just prior to ingestion. The kits may comprise a first container comprising soluble alginate; and second container comprising insoluble alginate/gelling ion particles. The individual containers may be separate container compartments of an integrated container system.

The multi-component forms of the edible product may be presented in the form of a kit. In some embodiments, the kits comprise polysaccharide in the form of a solution. In some embodiments, the kits may comprise polysaccharide free of a solvent. In some embodiments, the kits may comprise an additional container comprising a solvent, e.g. water, milk, or some other form of beverage.

In some embodiments, the kits may comprise the source of multivalent cation in the form of a powder or granulates. In some embodiments, the kits may comprise the source of multivalent cation in the form of a dispersion.

In some embodiments, the kits may comprise an additional container comprising pharmaceutical actives, nutrients, nutraceuticals, vitamins, minerals, proteins, probiotics and prebiotics. In some embodiments, the kits comprise pharmaceutical actives, nutrients, nutraceuticals, vitamins, minerals, proteins, probiotics, and prebiotics in the container comprising soluble alginate solution or powder and/or in the container comprising the polysaccharide.

The different edible forms of the invention will require different packaging formats. For the dry powder reconstituted in water the presentation format could be a sachet, a box containing several servings, a bottle with the size of one serving with one portion powder mix designed to be filled with water and shaken before consumption, a two compartment package with one component in one part and water or solution in the other.

Liquid formulations may be presented in a two compartment package with a liquid formulation in one compartment and a powder, granulate or dispersion in the other. The liquid may contain all ingredients except one, e.g., the source of multivalent ions or the acidifying component. The two compartment package is preferably designed so that the barrier separating the two ingredients easily can be broken, pierced or removed to permit the liquid and powder to be mixed prior to consumption. One example is a two-compartment pouch wherein the powder and liquid are mixed when the pouch is squeezed and shaken just before consumption. In another example, a pouch or portion package can be provided wherein a bottom is pressed/turned so that one component is mixed with another component which may be a powder, liquid or a fizz tablet with an acidifying powder that may be added to the liquid phase just before consumption.

Compartmented containers for different types of materials may be employed. For example, a laminated package provided with an intermediate seal which includes a release area to allow two materials to intermix can be employed. Alternatively, internal type seals in a multi-compartment package may be used to separate the container contents until the seals are broken to mix the contents. Rupturable barriers may be used in such compartmented containers.

Other types of compartmented containers for separately storing ingredients that are later mixed within the container and consumed may be employed. For example, a dual container for separately storing two components having a main compartment containing a first beverage and a hollow cap for the main compartment which contains a second component. Mechanical means may be used to rupture the hollow cap and dispense the second component into the first. Alternatively, a dispensing lid for a beverage container may incorporate one or more frangible vessels containing one or more materials which are ordinarily added to popular beverages. Application of pressure to the top of the vessel may be used to rupture the underside and dispenses the contents into the beverage container. A container for a food may be provided with a cover with one or more frangible compartments. The compartments may contain a component which is intended to be added to the food in the container. As the cover is removed from the container, the compartment(s) are ruptured and the contents thereof may be automatically dispensed into the container. Alternatively, a cap for a beverage container including a compartment that holds a material to be added to the beverage. As the cap is removed, the compartment is automatically opened and the contents are dispensed into the beverage container. Various conventional container designs for separately storing a beverage or the like and an ingredient to be added thereto may be used in the implementation of the present invention.

Another approach for adding a desired amount of a solid material to a beverage involves the use of a solid material in the form of a stick that is used to stir the beverage. The more the beverage is stirred, the more solid material is dissolved. A variation on this approach is use of a stirring stick that stores a solid within a compartment. The stick is manipulated to open the compartment in any suitable manner such that when the beverage is stirred, the solid material within the compartment is dissolved in the beverage.

In another variation, one component of the material may be provided in the form of sprinkles that can be added to a liquid material and mixed therewith. Also, containers having two separate compartments that are exposed by opening the container may be employed. In this embodiment, the consumer has to move one component from its compartment to the other compartment to mix the components. This is similar to the way yoghurt and fruit are frequently packaged together.

In another embodiment, the container is designed for mechanical actuation of means for adding the second component to the first component, by, for example, twisting, squeezing, pressing a button, turning a knob, pulling a pull tab or some other means. In this embodiment, some manipulation by the consumer mixes the components together and further mixing can be accomplished by, for example, shaking the container.

In another embodiment, the components of the composition of the invention can be presented in solid form such as in a food bar, cookie, cereal, etc. which is designed to be chewed prior to ingestion. This embodiment preferably employs at least two different components, one of which may be, for example, coated on the other, or the two components may be embodied in separate physical layers of the edible product. In this manner, the product can be hydrated in the oral cavity and/or stomach as it is chewed and/or swallowed.

The composition of the invention may be packaged with instructions and/or indicia which teach the use of the composition for weight loss and/or controlling appetite or food consumption. The indicia or instruction may be in any form, such as written instructions and pictorial representations.

The present invention is now described in more detail by reference to the following examples, but it should be understood that the invention is not construed as being limited thereto. Unless otherwise indicated herein, all parts, percents, ratios and the like are by weight.

### EXAMPLES

### Example 1

To determine the influence of the acidic component on gelation, two formulations were prepared according to Table 1. Each formulation contained a suitable concentration of an acid soluble calcium salt, such that upon solubilisation sufficient calcium ions would be liberated to cross-link and gel the sodium alginate. The formulation is a dry powder reconstitutable in water and the composition (%w/v) refers to the concentration of ingredient upon reconstitution.

**Table 1. Compositions of Formulations A and B used for gel strength testing.**

| Ingredient | Composition (%w/v) Formulation A | Composition (%w/v) Formulation B |
|---|---|---|
| Sodium alginate (Protanal LHS-1, FMC Biopolymer) | 1.5 | 1.5 |
| Calcium carbonate (HuberCAL, OSP 1000FG, Huber Engineered Materials | 0.25 | 0.25 |
| Glucono-delta-lactone (USP, Sigma-Aldrich) | 1.6 | - |
| Fructose (PhEur, Fluka) | 7 | 7 |

The dry powder was reconstituted in 100mL water. To determine how the reconstituted formulations behaved when in contact with a gastric fluid of reduced acidity, they were added to 50mL of modified USP simulated gastric fluid. The modified gastric fluid had been corrected to pH 5.5 and pre-heated to 37°C. Following mixing the formulation:gastric fluid mix was incubated at 37°C and the mechanical properties of the gel determined after 30 minutes. The mechanical properties of the gel were determined using a texture analyser (Model TA.XT Plus, Stable Micro Systems, UK, load cell capacity 5kg). The instrument applies a mechanical stress to a sample and measures the force profile associated with that stress. In this study the strength of the gel was assessed by its ability to withstand compression. The gel was placed under the texture analyser and a cylindrical delrin ½" probe applied to the surface of the gel and then lowered to a distance of 5mm at a speed of 0.5mm s⁻¹. Following removal of the probe the gel strength is assigned as the maximum peak force recorded during the compression cycle. The texture analyser has a threshold resistance to compression of 0.05N, if a sample does not offer a resistance to compression greater than this threshold a recordable value of gel strength cannot be obtained. The gel strength of five independently prepared samples of each formulation was determined.

Table 2 shows the gel strength of each formulation after addition to 50mL of a weak acid gastric fluid media. Formulation B, prepared without the acid component, did not gel in the gastric fluid media, whereas an identical formula containing the acid component (Formulation A) formed a gel with considerable mechanical strength. The data suggests that when a polysaccharide:acid soluble calcium salt formulation is added to a gastric fluid with reduced acidity, insufficient calcium ions are solubilised to gel the sodium alginate. The co-formulation of an acidic component enhances and in this case ensures gelation of the polysaccharide:acid soluble calcium salt formulation. Therefore including an acidic component in pharmaceutical compositions that rely on intra-gastric gelation provides an effective means of enhancing functionality.

**Table 2. Influence of the acid component on the gel strength of polysaccharide:acid soluble calcium salt formulations.**

| Formulation | Gel Strength (N) |
|---|---|
| | Mean (n=5) ± 1SD |
| Formulation A | 30±1.5 |
| Formulation B | <0.05 |

### Example 2

| **Dry Powder Reconstituted in Water** | |
|---|---|
| Sodium alginate (Protanal LHS-1, FMC BioPolymer) | 1.5g |
| Calcium carbonate (HuberCAL OSP 1000FG, Huber Engineered Materials) | 0.7g |
| Glucono-delta-lactone (USP, Sigma-Aldrich) | 2.8g |
| Malic acid (USP/NF Sigma-Aldrich) | 0.05g |
| Fructose (PhEur, Fluka) | 7g |
| Sodium bicarbonate (PhEur, Fluka) | 0.5g |
| Flavour (Vanilla) (prod. No. 10981-31, Givaudan Schweiz AG) | 0.24g |
| Water added on reconstitution | to 100mL |

A reconstitutable dry powder to be mixed with 100mL of water. Dry blend all ingredients and package accordingly. The composition remains as a low viscosity drinkable liquid for 10 minutes. Upon ingestion it forms a substantial gelatinous mass in the stomach with a volume of up to 200cm³. The liquid formulation should be taken prior to meals and may be used in the prevention/management of overweight/obesity or as the base formula for a meal replacement product. The intra-gastric formation of a voluminous mass may induce satiety by initiating the physiological pathways involved in appetite suppression.

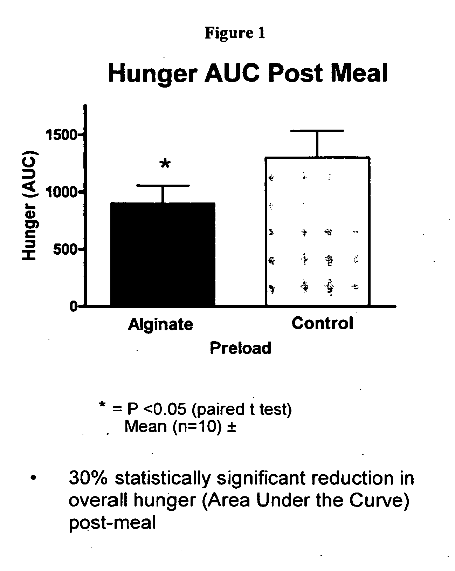

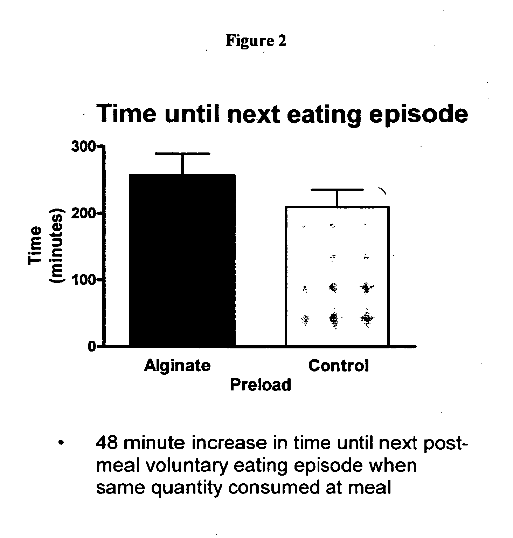

Clinical testing of the formulation above using robust methodologies (reviewed by Reid & Hetherington) has been executed on a test panel of 10 volunteers. The test was a randomized, controlled, single-blind two-way crossover trial to measure food consumption, relative fullness and relative hunger. The study had a three day follow up on two occasions. Subjects completed a visual analogue scale (VAS) questionnaire before, during and after the test meal. The preload composition was taken 30 minutes before lunch. The relative hunger, post-meal, is given in Figure 1 and the time until the next eating episode is given in Figure 2.

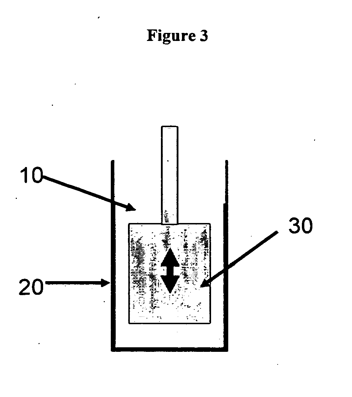

The gelation kinetics of the composition of Example 2 was then investigated. The gelation kinetics was determined using an apparatus as shown in Figure 3. Formulation 10 is located in a vessel 20 provided with a cylindrical probe 30. Cylindrical probe 30 is oscillated in formulation 10 and attached to a load cell to allow the load to be measured to provide an indication of the gel strength of the material. This permits detection of resistance to the movement of cylindrical probe 30 and thus degree of gelation in a non-destructive manner.

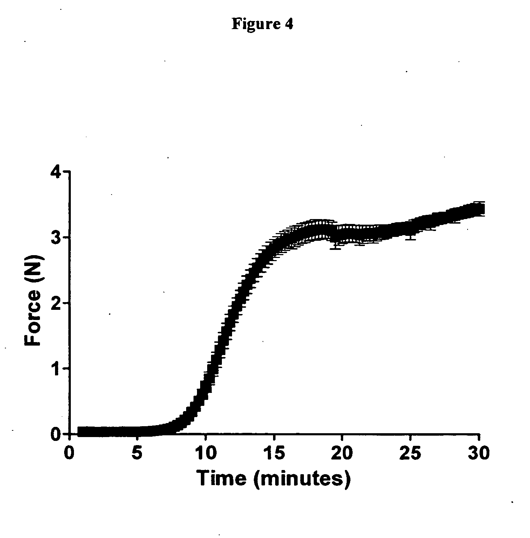

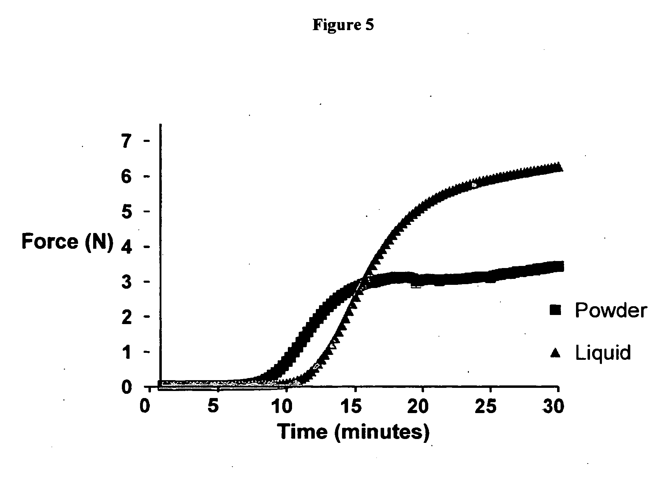

A powder formulation containing all ingredients given above in the table was mixed with 100ml of water and shaken for 10 seconds to prepare a first sample for testing (hereinafter "the Powder Formulation"). A liquid formulation containing all ingredients except the glucono-delta lactone was decanted into a plastic bottle, the glucono-delta lactone component was added and the mixture was shaken for 10 seconds to prepare a second sample for testing (hereinafter, "the Liquid Formulation"). The results for the Powder Formulation are shown in Figure 4 and a comparison of the results for the Powder and Liquid Formulations is shown in Figure 5.

The results show that the Liquid Formulation exhibited a longer time until significant gelation occurred and formed a stronger gel after about 30 minutes.

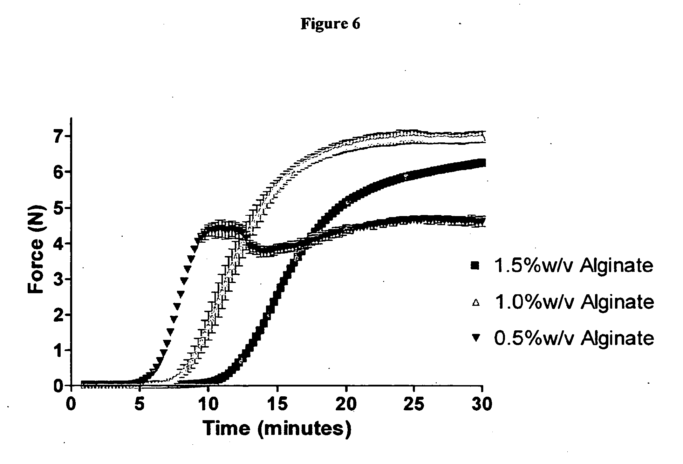

Additional examples were conducted using the same formulation as given above, except with variations in the concentration of alginate employed. The results of these additional examples with varying amounts of alginates are shown in Figure 6. Increasing the alginate concentration reduced the time until significant gelation occurred and influenced the final gel strength of the gelled composition.

### Example 3

| **Dry Powder Reconstituted in Water** | |
|---|---|
| Sodium alginate (Protanal LHS-1, FMC BioPolymer) | 1.5g |
| Calcium carbonate (HuberCAL OSP 1000FG, Huber*) | 0.7g |
| Glucono-delta-lactone (USP,Sigma-Aldrich) | 2.8g |
| Fructose (PhEUR, Fluka) | 7g |
| Sodium bicarbonate (PhEur, Fluka) | 0.44g |
| Flavour (Vanilla) (Prod.no.1 0981-31, Givaudan Schweiz AG) | 0.24g |
| Water added on reconstitution | to 100mL |

| | |
|---|---|
| *Huber Engineered Materials | |

A reconstitutable dry powder designed to be mixed with 100mL of water is listed in the table above. All ingredients are to be dry blended and packaged accordingly. The composition remains as a low viscosity drinkable liquid for 10 minutes. Upon ingestion it forms a substantial gelatinous mass in the stomach with a volume of up to 200cm³. The liquid formulation should be taken prior to meals and may be used in the prevention/management of overweight/obesity or as the base formula for a meal replacement product. The intra-gastric formation of a voluminous mass may induce satiety by initiating the physiological pathways involved in appetite suppression.

### Example 4

| **Dry Powder Reconstituted in Milk** | |
|---|---|
| Sodium alginate (Protanal LHS-1, FMC BioPolymer) | 1.5g |
| Calcium carbonate (HuberCAL OSP 1000FG, Huber*) | 0.7g |
| Glucono-delta-lactone (USP,Sigma-Aldrich) | 2.8g |
| Malic acid (USP/NF Sigma-Aldrich) | 0.05g |
| Fructose (PhEUR, Fluka) | 7g |
| Sodium bicarbonate (PhEur, Fluka) | 0.5g |
| Flavour (Vanilla) (Prod.no.10981-31, Givaudan Schweiz AG) | 0.24g |
| Milk added on reconstitution | to 100mL |

| | |
|---|---|
| *Huber Engineered Materials | |

A reconstitutable dry powder designed to be mixed with 100mL of milk. Dry blend all ingredients and package accordingly. The composition remains as a low viscosity drinkable liquid for 10 minutes. Upon ingestion, the composition forms a substantial gelatinous mass in the stomach with a volume of up to 200cm³. The liquid formulation should be taken prior to meals and may be used in the prevention/management of overweight/obesity or as the base formula for a meal replacement product. The intra-gastric formation of a voluminous mass may induce satiety by initiating the physiological pathways involved in appetite suppression.

### Example 5

| **Ready to drink formulation** | |
|---|---|
| Sodium alginate (Protanal LHS-1, FMC BioPolymer) | 1.5g |
| Calcium carbonate (HuberCAL OSP 1000FG, Huber*) | 0.7g |
| Fructose (PhEUR, Fluka) | 7g |
| Sodium bicarbonate (PhEur, Fluka) | 0.44g |
| Flavour (Vanilla) (Prod.no.10981-31, Givaudan Schweiz AG) | 0.24g |
| Water added on reconstitution | to 100mL |
| Glucono-delta-lactone | 2.8g |

Dissolve Sodium alginate, calcium carbonate, fructose and flavour in 100mL of water. Glucono-delta-lactone is to be added to the viscous solution and shaken just before consumption. The product will be presented in a two compartment package wherein the liquid phase and the dry powder (glucono-delta-lactone-containing component) are kept apart until consumption. This package can be a pouch with two compartments where the pouch may be squeezed to break a barrier between the compartments and shaken to mix the powder into the liquid phase. The composition remains as a low viscosity drinkable liquid for about 10 minutes. Upon ingestion it forms a substantial gelatinous mass in the stomach with a volume of up to 200cm³. The liquid formulation should be taken prior to meals and may be used in the prevention/management of overweight/obesity or as the base formula for a meal replacement product. The intra-gastric formation of a voluminous mass may induce satiety by initiating the physiological pathways involved in appetite suppression. The composition can also be formulated without sodium bicarbonate but the volume of the gelatinous mass in the stomach after ingestion will then be less.

### Example 6

| **Ready to drink formulation** | |
|---|---|
| Sodium alginate (Protanal LF 5/60, FMC BioPolymer) | 2.5g |
| Calcium carbonate (HuberCAL OSP 1000FG, Huber*) | 1.17g |
| Fructose (PhEur, Fluka) | 11.7g |
| Sodium bicarbonate (PhEur, Fluka) | 0.73g |
| Flavour (Vanilla) (Prod.no.10981-31, Givaudan Schweiz AG) | 0.40g |
| Water added on reconstitution | to 50 mL |
| Glucono-delta-lactone | 2.8g |

| | |
|---|---|
| * Huber Engineered Materials | |

Dissolve sodium alginate, calcium carbonate, fructose and flavour in 50mL of water. Glucono-delta-lactone is to be added to the viscous solution and shaken just before consumption. The product will be presented in a two compartment package where the liquid phase and the dry powder (containing the glucono-delta-lactone) are kept apart until consumption. This package can be a pouch with two compartments where the pouch is squeezed to break a barrier between the compartments and shaken to mix the powder into the liquid phase. The composition remains as a low viscosity drinkable liquid for 10 minutes. Upon ingestion, the composition forms a substantial gelatinous mass in the stomach with a volume of up to 100cm³. The liquid formulation should be taken prior to meals and may be used in the prevention/management of overweight/obesity or as the base formula for a meal replacement product. The intra-gastric formation of a voluminous mass may induce satiety by initiating the physiological pathways involved in appetite suppression. The composition can also be without sodium bicarbonate added but the volume of the gelatinous mass in the stomach after ingestion will then be slightly less.

### Example 7

| **Ready to drink formulation** | |
|---|---|
| Sodium alginate (Protanal LHS-1, FMC BioPolymer) | 1.5g |
| Calcium carbonate (HuberCAL OSP 1000FG, Huber*) | 0.7g |
| Fructose (PhEUR, Fluka) | 7g |
| Sodium bicarbonate (PhEur, Fluka) | 0.44g |
| Flavour (Vanilla) (Prod.no.10981-31, Givaudan Schweiz AG) | 0.24g |
| Water added on reconstitution | to 300mL |
| Glucono-delta-lactone | 2.8g |

| | |
|---|---|
| * Huber Engineered Materials | |

Dissolve sodium alginate, calcium carbonate, fructose and flavour in 300mL of water. Glucono-delta-lactone is to be added to the viscous solution and shaken just before consumption. The product will be presented in a two compartment package where the liquid phase and the dry powder (containing glucono-delta-lactone) are kept apart until consumption. This package can be a pouch with two compartments where the pouch is squeezed to break a barrier between the compartments and shaken to mix the powder into the liquid phase. The composition remains as a low viscosity drinkable liquid for 10 minutes. Upon ingestion, the composition forms a substantial gelatinous mass in the stomach with a volume of up to 400cm³. The liquid formulation should be taken prior to meals and may be used in the prevention/management of overweight/obesity or as the base formula for a meal replacement product. The intra-gastric formation of a voluminous mass may induce satiety by initiating the physiological pathways involved in appetite suppression. The composition can also be without sodium bicarbonate added but the volume of the gelatinous mass in the stomach after ingestion will then be less.

### Example 8

| **Dry Powder Reconstituted in Water** | |
|---|---|
| Levodopa (Sigma-Aldrich) | 500mg |
| Sodium alginate (Protanal SF200,FMC BioPolymer) | 2g |
| Calcium carbonate (HuberCAL OSP 1000FG, Huber*) | 0.93g |
| Glucono-delta-lactone (USP,Sigma-Aldrich) | 3.0g |
| Fumaric acid (USP/NF, Sigma-Aldrich) | 0.05g |
| Fructose (PhEUR, Fluka) | 10g |
| Sodium bicarbonate (PhEur, Fluka) | 0.6g |
| Flavour (Vanilla) (Prod.no.10981-31, Givaudan Schweiz AG) | 0.24g |
| Water added on reconstitution | to 100ml |

A reconstitutable dry powder to be mixed with 100mL of water is described in the table above. Dry blend all ingredients and package accordingly. The composition remains as a low viscosity drinkable liquid for 10 minutes. Upon ingestion, the composition forms a substantial gelatinous mass in the stomach with a volume of up to 200cm³. A mass of this size and rigidity can be retained in the stomach as it will require mechanical erosion to pass through the pyloric sphincter. The liquid formulation may therefore be used as a gastro-retentive dosage form for delivering drugs with specific absorption windows in the upper gastro-intestinal tract or for localised treatment of upper gastro-intestinal conditions.

### Example 9 (not according to the invention)

| **Granules Reconstituted in Water** | |
|---|---|
| Sodium alginate (Protanal LFR 5/60) | 1g |
| Pectin (Unipectine 903) | 3g |
| Calcium carbonate | 0.53g |
| Colloidal silica | 0.04g |
| Fumaric acid | 2g |
| Sodium bicarbonate | 0.66g |
| Fructose | 9g |
| Flavour (Vanilla) | 0.24g |
| Water added on reconstitution | to 100mL |

Dry blend all of the ingredients excluding the fumaric acid, fructose, flavour and 0.4g sodium bicarbonate. Granulate using a solution of 70:30% v/v ethanol:water and dry at 50°C. Pass the granules through a 1000 µm sieve. Dry blend the excluded ingredients with the granules and package accordingly. A reconstitutable granulated product to be mixed with 100mL of water is obtained. The granules should be ingested after meals and may be used in the prevention/management of overweight/obesity. The intra-gastric gelation of the formulation encapsulates/viscosifies the ingested meal and may modulate nutrient absorption.

### Example 10

| **Sustained release tablet** | |
|---|---|
| Celiprolol hydrochloride | 400mg |
| Sodium alginate | 100mg |
| Calcium hydrogen phosphate | 420mg |
| Magnesium stearate | 20mg |
| Titanium dioxide | 100mg |
| Glucono-delta-lactone | 150mg |

Dry blend all of the ingredients excluding the glucono-delta-lactone. Wet granulate using a solution of 70:30% v/v ethanol:water and dry at 50°C. Pass the granules through a 1000 µm sieve then dry blend with the glucono-delta-lactone. Compress using a conventional tablet press.

### Example 11

| **Anti-Reflux Liquid** | |
|---|---|
| Sodium alginate (LFR 5/60) | 50.0g |
| Calcium carbonate | 16.0g |
| Glucono-delta-lactone coated in a thermolabile protectant | 90g |
| Sodium bicarbonate | 26.7g |
| Methyl parahydroxybenzoate | 4.0g |
| Propyl parahydroxybenzoate | 0.6g |
| Carbopol 974P | 1.0g |
| Sodium hydroxide | 0.46g |
| Sodium saccharin | 1.0g |
| Flavour | 0.7g |
| Deionised water | to 1000mL |

The Carbopol 974P is dispersed in 450mL deionised water and neutralised with the sodium hydroxide. The glucono-delta-lactone, sodium bicarbonate, calcium carbonate, parahydroxybenzoates and saccharin are mixed in a separate second vessel with 450mL deionised water. The sodium alginate is added slowly to the second vessel until it is fully hydrated. The sodium alginate phase in then added to the Carbopol phase and mixed until fully dispersed. The flavour is added and stirred in.

The volume is adjusted to 1000mL using deionised water and stirred until fully mixed. The glucono-delta-lactone (GDL) is coated in a thermo-labile coating to prevent premature acidification of the anti-reflux liquid. Following ingestion the GDL is released in the stomach as the anti-reflux liquid adjusts to body temperature. It then becomes available to ensure/enhance intra-gastric gelation.

### Example 12

| **Anti-Reflux Tablet** | | |
|---|---|---|
| 1 | Sodium alginate (LHS-1, FMC BioPolymer) | 250g |
| 2 | Calcium carbonate (HuberCAL, OSP 1000FG, Huber*) | 80g |
| 3 | Glucono-delta-lactone (USP, Sigma-Aldrich) | 450g |
| 4 | Sodium bicarbonate (PhEur, Fluka) | 133.5g |
| 5 | Mannitol (C*PharmMannidex DC (200 grade) 16702, Cerestar UK) | 522.75g |
| 6 | Crospovidone (Kollidon CL, BASF) | 55g |
| 7 | Magnesium stearate (Fisher Scientific UK) | 15g |
| 8 | Flavour | 6g |
| 9 | Acesulfame (Nutrinova UK) | 5.5g |
| 10 | Aspartame (Holland Sweetener Company) | 1.65g |

| | | |
|---|---|---|
| *Huber Engineered Materials | | |

Dry blend ingredients 1,2 and 4 in a high shear mixer. Wet granulate using a solution of 70:30%v/v ethanol:water, dry at 50°C. Mill the granules through a 610µm screen and secondly a 457µm screen using a Quadro Comill, then dry blend with ingredients 3, 5, 6, 8, 9 and 10 in a low shear tumble mixer for 5 minutes. Add ingredient 7 and continue blending for 2 minutes. Compress using a conventional tablet press and package accordingly.

### Example 13

Sodium alginate and calcium carbonate can be incorporated in a pH neutral spoonable dessert such as a soy based chocolate cream. A powder acidifier is sprinkled into the dessert just before consumption. This dessert would be presented in a two-compartment package with the spoonable dessert in the main compartment and the powder acidifier in a smaller, secondary compartment. An acidifier such as glucono-delta-lactone may be provided in powder form, and may be mixed with other ingredients such as dry chocolate beads, sugar, muesli, etc. An alternative is to provide an acidified spoonable dessert like a fruit puree in the main compartment and a calcium carbonate containing-component with a protective coating in a secondary compartment. Just prior to ingestion the contents of the secondary compartment is added into the main compartment and mixed by stirring, whereby gelling of the polysaccharide is initiated. Upon ingestion, the formulation forms a substantial gelatinous mass in the stomach with a volume. The formulation should be taken prior to meals and may be used in the prevention/management of overweight/obesity. The intra-gastric formation of a voluminous mass may induce satiety.

### Example 14

Sodium alginate and calcium carbonate is mixed with other dry ingredients to prepare an edible food bar. A powder acidifier such as glucono-delta-lactone can be sprinkled on an exterior surface of the food bar. Upon ingestion, a substantial gelatinous mass forms in the stomach. The food bar should be taken prior to meals and may be used in the prevention/management of overweight/obesity or as the base formula for a meal replacement product. During and after ingestion, the alginate will partly form a gel network in the stomach due to release of calcium ions from calcium carbonate as a result of acidification by the combination of stomach acid and the acidifier. The intra-gastric formation of a voluminous mass may induce satiety due to gastric stretch and slow emptying of the stomach contents due to the presence of the gelled structure.

## Claims

1. A cosmetic treatment method for inducing satiety in mammals, comprising the step of ingesting a composition comprising:
(a) an alginate or alginate salt capable of ionotropic gelation,
(b) at least one source of multivalent cations selected from salts of calcium, iron, strontium, barium, chromium, manganese, nickel, cobalt, molybdenum, copper, aluminum and zinc, which is capable of solubilization at an acidic pH, and
(c) a lactone of a carboxylic acid capable of providing a controlled release of protons sufficient to solubilize said multivalent cations.

2. The method of claim 1, wherein said composition is a liquid and is ingested by drinking.

3. The method of claim 1, wherein said composition is a solid and is hydrated in the oral cavity when ingested.

4. The method of claim 1, wherein said composition is formed by the step of mixing a solid and a liquid to thereby combine components (a)-(c) in a hydrating environment prior to ingestion of said composition.

5. The method of any one of claims 1-4, wherein the composition further comprises a dispersing agent.

6. The method of claim 5, wherein said dispersing agent is a solid.

7. The method of claim 6, wherein said solid is a crystalline sugar and at least one of sorbitol, fructose, polydextrose, sucrose, maltose, maltitol, xylitol or mannitol.

8. The method of claim 7, wherein said crystalline sugar is fructose.

9. The method of claim 7, wherein said solid is a protein.

10. The method of claim 5, wherein dispersing agent is a liquid.

11. The method of any one of claims 1-10, wherein said composition further comprises a gas forming component.

12. The method of claim 11, wherein said gas forming component is selected from sodium bicarbonate, potassium bicarbonate, ammonium bicarbonate, and calcium carbonate.

13. The method of claim 12, wherein said gas forming component is sodium bicarbonate.

14. The method of claim 12, wherein said gas forming component is potassium bicarbonate.

15. The method of any one of claims 12-14, wherein said composition further comprises up to 0.5% by weight of an acid component capable of providing immediate release of protons.

16. The method of claim 15, wherein said acid capable of providing immediate release of protons is malic acid.

17. The method of any one of claims 1-16, wherein said component (a) is at least one of a sodium alginate or potassium alginate.

18. The method of any one of claims 1-16, wherein said component (a) is alginate.

19. The method of any one of claims 1-16, wherein said component (a) is potassium alginate.

20. The method of any one of claims 1-16, wherein said component (a) is sodium alginate.

21. The method of any one of claims 1-20, wherein said acid component capable of providing a controlled release of protons is glucono-delta-lactone.

22. The method of any one of claims 1-21, wherein the mole ratio of component (a) to component (b) is less than 30:1.

23. The method of any one of claims 1-21, wherein the mole ratio of component (a) to component (b) is less than 22:1.

24. A method of any one of claims 1-21, wherein the mole ratio of component (a) to component (b) is less than 6:1.

25. The method of any one of claims 1-24, wherein the mole ratio of acid moieties of component (c) to a number of moles of component (a) is more than 1:1.

26. The method of any one of claims 1-24, wherein the mole ratio of acid moieties of component (c) to a number of moles of component (a) is more than 10:1.

27. The method of any one of claims 1-24, wherein the mole ratio of acid moieties of component (c) to a number of moles of component (a) is more than 100:1.

28. The method of any one of claims 1-27, wherein an amount of component (a) administered per meal is 0.5-5.0 grams.

29. The method of any one of claims 1-27, wherein an amount of component (a) administered per meal is 0.75-3.0 grams.

30. A composition comprising:
(a) an alginate or alginate salt capable of ionotropic gelation,
(b) at least one source of multivalent cations selected from salts of calcium, iron, strontium, barium, chromium, manganese, nickel, cobalt, molybdenum, copper, aluminum and zinc, which is capable of solubilization at an acidic pH,
(c) a lactone of a carboxylic acid capable of providing a controlled release of protons sufficient to solubilize said multivalent cations, and
(d) a gas forming component,
said composition is capable of hydrating in aqueous media and subsequently forming a gel in the stomach when ingested that resists peristaltic forces and remains in the stomach for an extended time.

31. a composition comprising:
(a) an alginate or alginate salt capable of ionotropic gelation,
(b) at least one source of multivalent cations selected from salts of calcium, iron, strontium, barium, chromium, manganese, nickel, cobalt, molybdenum, copper, aluminum and zinc, which is capable of solubilization at an acidic pH, and
(c) a lactone of a carboxylic acid capable of providing a controlled release of protons sufficient to solubilize said multivalent cations for use for treatment of obesity.

32. The composition of claim 31, further comprising a gas forming component.

33. The composition of any one of claims 30-32, further comprising a dispersing agent.

34. The composition of claim 33, wherein said dispersing agent is a solid.

35. The composition of claim 34, wherein said solid is a crystalline sugar and at least one of sorbitol, fructose, polydextrose, sucrose, maltose, maltitol, xylitol or mannitol.

36. The composition of claim 35, wherein said crystalline sugar is fructose.

37. The composition of claim 34, wherein said solid is a protein.

38. The composition of claim 33, wherein dispersing agent is a liquid.

39. The composition of any one of claims 30 and 32-37, wherein said gas forming component is selected from sodium bicarbonate, potassium bicarbonate, ammonium bicarbonate, and calcium carbonate.

40. The composition of claim 39, wherein said gas forming component is sodium bicarbonate.

41. The composition of claim 39, wherein said gas forming component is potassium bicarbonate.

42. The composition of any one of claims 30-41, further comprising up to 0.5% by weight of an acid component capable of providing immediate release of protons.

43. The composition of claim 42, wherein said acid capable of providing immediate release of protons is malic acid.

44. The composition of any one of claims 30-43, further comprising at least one active ingredient selected from: pharmaceutical actives, nutrients, nutraceuticals, vitamins, minerals, proteins, probiotics, and prebiotics.

45. The composition of any one of claims 30-44, wherein said component (a) is alginate.

46. The composition of any one of claims 30-44, wherein said component (a) is sodium alginate.

47. The composition of any one of claims 30-44, wherein said component (a) is potassium alginate.

48. The composition of any one of claims 30-44, wherein said component (a) is a combination of sodium alginate and low-methoxylated sodium pectate.

49. The composition of any one of claims 30-44, wherein said component (a) is a combination of potassium alginate and low-methoxylated potassium pectate.

50. The composition of any one of claims 30-44, wherein said component (a) is alginate in combination with an iota carrageenan.

51. The composition of any one of claims 30-50, wherein said acid component capable of providing a controlled release of protons is glucono-delta-lactone.

52. A composition as claimed in any one of claims 30-51, wherein the mole ratio of component (a) to component (b) is less than 30:1.

53. A composition as claimed in any one of claims 30-51, wherein the mole ratio of component (a) to component (b) is less than 22:1.

54. A composition as claimed in any one of claims 30-51, wherein the mole ratio of component (a) to component (b) is less than 6:1.

55. A composition as claimed in any one of claims 30-54, wherein the mole ratio of acid moieties of component (c) to a number of moles of component (a) is more than 1:1.

56. A composition as claimed in any one of claims 30-54, wherein the mole ratio of acid moieties of component (c) to a number of moles of component (a) is more than 10:1.

57. A composition as claimed in any one of claims 30-54, wherein the mole ratio of acid moieties of component (c) to a number of moles of component (a) is more than 100:1.

## Patentansprüche

1. Eine kosmetische Behandlungsmethode zur Induzierung von Sättigung in Säugetieren, umfassend den Schritt der Aufnahme einer Zusammensetzung, die Folgendes umfasst:
(a) ein Alginat oder Alginatsalz fähig zur ionotropen Gelierung,
(b) mindestens eine Quelle multivalenter Kationen ausgewählt unter Salzen von Calcium, Eisen, Strontium, Barium, Chrom, Mangan, Nickel, Kobalt, Molybdän, Kupfer, Aluminium und Zink, welche in der Lage ist, bei saurem pH zu solubilisieren, und
(c) ein Lacton einer Carboxylsäure fähig zur Bereitstellung einer kontrollierten Freisetzung von Protonen ausreichend zum Solubilisieren besagter multivalenter Kationen.

2. Die Methode gemäß Anspruch 1, wobei besagte Zusammensetzung eine Flüssigkeit ist und durch Trinken aufgenommen wird.

3. Die Methode gemäß Anspruch 1, wobei besagte Zusammensetzung ein Festkörper ist und bei Aufnahme in der Mundhöhle hydratisiert wird.

4. Die Methode gemäß Anspruch 1, wobei besagte Zusammensetzung durch den Schritt des Mischens eines Festkörpers und einer Flüssigkeit gebildet wird, um so die Komponenten (a)-(c) vor Aufnahme besagter Zusammensetzung in einer hydratisierenden Umgebung zu kombinieren.

5. Die Methode gemäß irgendeinem der Ansprüche 1-4, wobei die Zusammensetzung ferner ein Dispergiermittel enthält.

6. Die Methode gemäß Anspruch 5, wobei besagtes Dispergiermittel ein Festkörper ist.

7. Die Methode gemäß Anspruch 6, wobei besagter Festkörper ein Kristallzucker und mindestens einer von Sorbitol, Fructose, Polydextrose, Sucrose, Maltose, Maltitol, Xylitol oder Mannitol ist.

8. Die Methode gemäß Anspruch 7, wobei besagter Kristallzucker Fructose ist.

9. Die Methode gemäß Anspruch 7, wobei besagter Festkörper ein Protein ist.

10. Die Methode gemäß Anspruch 5, wobei das Dispergiermittel eine Flüssigkeit ist.

11. Die Methode gemäß irgendeinem der Ansprüche 1-10, wobei besagte Zusammensetzung ferner eine gasbildende Komponente umfasst.

12. Die Methode gemäß Anspruch 11, wobei besagte gasbildende Komponente ausgewählt wird unter Natriumbicarbonat, Kaliumbicarbonat, Ammoniumbicarbonat und Calciumcarbonat.

13. Die Methode gemäß Anspruch 12, wobei besagte gasbildende Komponente Natriumbicarbonat ist.

14. Die Methode gemäß Anspruch 12, wobei besagte gasbildende Komponente Kaliumbicarbonat ist.

15. Die Methode gemäß irgendeinem der Ansprüche 12-14, wobei besagte Zusammensetzung ferner bis zu 0,5 Gew.-% einer Säurekomponente umfasst, welche zur Bereitstellung einer sofortigen Freisetzung von Protonen fähig ist.

16. Die Methode gemäß Anspruch 15, wobei besagte zur Bereitstellung einer sofortigen Freisetzung von Protonen fähige Säure Äpfelsäure ist.

17. Die Methode gemäß irgendeinem der Ansprüche 1-16, wobei besagte Komponente (a) mindestens eines von Natriumalginat oder Kaliumalginat ist.

18. Die Methode gemäß irgendeinem der Ansprüche 1-16, wobei besagte Komponente (a) Alginat ist.

19. Die Methode gemäß irgendeinem der Ansprüche 1-16, wobei besagte Komponente (a) Kaliumalginat ist.

20. Die Methode gemäß irgendeinem der Ansprüche 1-16, wobei besagte Komponente (a) Natriumalginat ist.

21. Die Methode gemäß irgendeinem der Ansprüche 1-20, wobei besagte zur Bereitstellung einer kontrollierten Freisetzung von Protonen fähige Säurekomponente Glucono-delta-lacton ist.

22. Die Methode gemäß irgendeinem der Ansprüche 1-21, wobei das Molverhältnis von Komponente (a) zu Komponente (b) weniger als 30:1 beträgt.

23. Die Methode gemäß irgendeinem der Ansprüche 1-21, wobei das Molverhältnis von Komponente (a) zu Komponente (b) weniger als 22:1 beträgt.

24. Die Methode gemäß irgendeinem der Ansprüche 1-21, wobei das Molverhältnis von Komponente (a) zu Komponente (b) weniger als 6:1 beträgt.

25. Die Methode gemäß irgendeinem der Ansprüche 1-24, wobei das Molverhältnis von Säureanteilen von Komponente (c) zu einer Anzahl von Molen von Komponente (a) mehr als 1:1 beträgt.

26. Die Methode gemäß irgendeinem der Ansprüche 1-24, wobei das Molverhältnis von Säureanteilen von Komponente (c) zu einer Anzahl von Molen von Komponente (a) mehr als 10:1 1 beträgt.

27. Die Methode gemäß irgendeinem der Ansprüche 1-24, wobei das Molverhältnis von Säureanteilen von Komponente (c) zu einer Anzahl von Molen von Komponente (a) mehr als 100:1 1 beträgt.

28. Die Methode gemäß irgendeinem der Ansprüche 1-27, wobei eine pro Mahlzeit verabreichte Menge von Komponente (a) 0,5-5,0 Gramm beträgt.

29. Die Methode gemäß irgendeinem der Ansprüche 1-27, wobei eine pro Mahlzeit verabreichte Menge von Komponente (a) 0,75-3,0 Gramm beträgt.

30. Eine Zusammensetzung, welche Folgendes umfasst:
(a) ein Alginat oder Alginatsalz fähig zur ionotropen Gelierung,
(b) mindestens eine Quelle multivalenter Kationen ausgewählt unter Salzen von Calcium, Eisen, Strontium, Barium, Chrom, Mangan, Nickel, Kobalt, Molybdän, Kupfer, Aluminium und Zink, welche in der Lage ist, bei saurem pH zu solubilisieren, und
(c) ein Lacton einer Carboxylsäure fähig zur Bereitstellung einer kontrollierten Freisetzung von Protonen ausreichend zum Solubilisieren besagter multivalenter Kationen, und
(d) eine gasbildende Komponente,
wobei besagte Zusammensetzung zum Hydratisieren in wässrigen Medien und bei Aufnahme zur nachfolgenden Bildung eines Gels im Magen fähig ist, welches peristaltischen Kräften widersteht und für einen längeren Zeitraum im Magen verbleibt.

31. Eine Zusammensetzung, welche Folgendes umfasst:
(a) ein Alginat oder Alginatsalz fähig zur ionotropen Gelierung,
(b) mindestens eine Quelle multivalenter Kationen ausgewählt unter Salzen von Calcium, Eisen, Strontium, Barium, Chrom, Mangan, Nickel, Kobalt, Molybdän, Kupfer, Aluminium und Zink, welche in der Lage ist, bei einem sauren pH zu solubilisieren, und
(c) ein Lacton einer Carboxylsäure fähig zur Bereitstellung einer kontrollierten Freisetzung von Protonen ausreichend zum Solubilisieren besagter multivalenter Kationen zum Gebrauch bei der Behandlung von Adipositas.

32. Die Zusammensetzung gemäß Anspruch 31 ferner umfassend eine gasbildende Komponente.

33. Die Zusammensetzung gemäß irgendeinem der Ansprüche 30-32 ferner umfassend ein Dispergiermittel.

34. Die Zusammensetzung gemäß Anspruch 33, wobei besagtes Dispergiermittel ein Festkörper ist.

35. Die Zusammensetzung gemäß Anspruch 34, wobei besagter Festkörper ein Kristallzucker und mindestens einer von Sorbitol, Fructose, Polydextrose, Sucrose, Maltose, Maltitol, Xylitol oder Mannitol ist.

36. Die Zusammensetzung gemäß Anspruch 35, wobei besagter Kristallzucker Fructose ist.

37. Die Zusammensetzung gemäß Anspruch 34, wobei besagter Festkörper ein Protein ist.

38. Die Zusammensetzung gemäß Anspruch 33, wobei das Dispergiermittel eine Flüssigkeit ist.

39. Die Zusammensetzung gemäß irgendeinem der Ansprüche 30 und 32-37, wobei besagte gasbildende Komponente ausgewählt wird unter Natriumbicarbonat, Kaliumbicarbonat, Ammoniumbicarbonat und Calciumcarbonat.

40. Die Zusammensetzung gemäß Anspruch 39, wobei besagte gasbildende Komponente Natriumbicarbonat ist.

41. Die Zusammensetzung gemäß Anspruch 39, wobei besagte gasbildende Komponente Kaliumbicarbonat ist.

42. Die Zusammensetzung gemäß irgendeinem der Ansprüche 30-41 ferner umfassend bis zu 0,5 Gew.-% einer Säurekomponente, welche zur Bereitstellung einer sofortigen Freisetzung von Protonen fähig ist.

43. Die Zusammensetzung gemäß Anspruch 42, wobei besagte zur Bereitstellung einer sofortigen Freisetzung von Protonen fähige Säure Äpfelsäure ist.

44. Die Zusammensetzung gemäß irgendeinem der Ansprüche 30-43 ferner umfassend mindestens einen wirksamen Bestandteil ausgewählt unter: Arzneimittelwirkstoffen, Nährstoffen, Nutrazeutika, Vitaminen, Mineralstoffen, Proteinen, Probiotika und Präbiotika.

45. Die Zusammensetzung gemäß irgendeinem der Ansprüche 30-44, wobei besagte Komponente (a) Alginat ist.

46. Die Zusammensetzung gemäß irgendeinem der Ansprüche 30-44, wobei besagte Komponente (a) Natriumalginat ist.

47. Die Zusammensetzung gemäß irgendeinem der Ansprüche 30-44, wobei besagte Komponente (a) Kaliumalginat ist.

48. Die Zusammensetzung gemäß irgendeinem der Ansprüche 30-44, wobei besagte Komponente (a) eine Kombination aus Natriumalginat und niedermethoxyliertem Natriumpektat ist.

49. Die Zusammensetzung gemäß irgendeinem der Ansprüche 30-44, wobei besagte Komponente (a) eine Kombination aus Kaliumalginat und niedermethoxyliertem Kaliumpektat ist.

50. Die Zusammensetzung gemäß irgendeinem der Ansprüche 30-44, wobei besagte Komponente (a) Alginat in Kombination mit einem Iota-Carrageen ist.

51. Die Zusammensetzung gemäß irgendeinem der Ansprüche 30-50, wobei besagte zur Bereitstellung einer kontrollierten Freisetzung von Protonen fähige Säurekomponente Glucono-delta-lacton ist.

52. Eine Zusammensetzung gemäß irgendeinem der Ansprüche 30-51, wobei das Molverhältnis von Komponente (a) zu Komponente (b) weniger als 30:1 beträgt.

53. Eine Zusammensetzung gemäß irgendeinem der Ansprüche 30-51, wobei das Molverhältnis von Komponente (a) zu Komponente (b) weniger als 22:1 beträgt.

54. Eine Zusammensetzung gemäß irgendeinem der Ansprüche 30-51, wobei das Molverhältnis von Komponente (a) zu Komponente (b) weniger als 6:1 beträgt.

55. Eine Zusammensetzung gemäß irgendeinem der Ansprüche 30-54, wobei das Molverhältnis von Säureanteilen von Komponente (c) zu einer Anzahl von Molen von Komponente (a) mehr als 1:1 beträgt.

56. Eine Zusammensetzung gemäß irgendeinem der Ansprüche 30-54, wobei das Molverhältnis von Säureanteilen von Komponente (c) zu einer Anzahl von Molen von Komponente (a) mehr als 10:1 1 beträgt.

57. Eine Zusammensetzung gemäß irgendeinem der Ansprüche 30-54, wobei das Molverhältnis von Säureanteilen von Komponente (c) zu einer Anzahl von Molen von Komponente (a) mehr als 100:1 1 beträgt.

## Revendications

1. Procédé de traitement cosmétique pour induire la satiété chez les mammifères, comprenant une étape d'ingestion d'une composition comprenant :
(a) un alginate ou un sel d'alginate apte à la gélation ionotropique,
(b) au moins une source de cations multivalents sélectionnés parmi les sels de calcium, de fer, de strontium, de baryum, de chrome, de manganèse, de nickel, de cobalt, de molybdène, de cuivre, d'aluminium, et de zinc, capable de se solubiliser sous pH acide, et
(c) une lactone d'acide carboxylique capable de fournir une libération contrôlée de protons en quantité suffisante pour solubiliser lesdits cations multivalents.

2. Procédé selon la revendication 1, **caractérisé en ce que** ladite composition est un liquide et est ingérée comme une boisson.

3. Procédé selon la revendication 1, **caractérisé en ce que** ladite composition est un solide et est hydraté dans la cavité orale lorsque ingéré.

4. Procédé selon la revendication 1, **caractérisé en ce que** ladite composition est formée dans une étape consistant à mélanger un solide avec un liquide pour ainsi obtenir les composants (a) à (c) dans un milieu hydrant avant ingestion de la dite composition.

5. Procédé selon l'une quelconque des revendications 1 à 4, **caractérisé en ce que** la composition comprend en outre un agent dispersant.

6. Procédé selon la revendication 5, **caractérisé en ce que** ledit agent dispersant est un solide.

7. Procédé selon la revendication 6, **caractérisé en ce que** ledit solide est un sucre cristallin et sélectionné parmi au moins le sorbitol, le fructose, le polydextrose, le sucrose, le maltose, le maltitol, le xylitol ou le mannitol.

8. Procédé selon la revendication 7, **caractérisé en ce que** ledit sucre cristallin est du fructose.

9. Procédé selon la revendication 7, **caractérisé en ce que** ledit solide est une protéine.

10. Procédé selon la revendication 5, **caractérisé en ce que** ledit agent dispersant est un liquide.

11. Procédé selon l'une quelconque des revendications 1 à 10, **caractérisé en ce que** ladite composition comprend en outre un composant gazogène.

12. Procédé selon la revendication 11, **caractérisé en ce que** ledit composant gazogène est sélectionné parmi le bicarbonate de sodium, le bicarbonate de potassium, le bicarbonate d'ammonium et le carbonate de calcium.

13. Procédé selon la revendication 12, **caractérisé en ce que** ledit composant gazogène est du bicarbonate de sodium.

14. Procédé selon la revendication 12, **caractérisé en ce que** ledit composant gazogène est du bicarbonate de potassium.

15. Procédé selon l'une quelconque des revendications 12 à 14, **caractérisé en ce que** ladite composition comprend en outre jusqu'à 0,5% par poids d'un composant acide capable de fournir une libération immédiate de protons.

16. Procédé selon la revendication 12, **caractérisé en ce que** ledit acide capable de fournir une libération immédiate de protons est de l'acide malique.

17. Procédé selon l'une quelconque des revendications 1 à 16, **caractérisé en ce que** ledit composant (a) est sélectionné parmi au moins un alginate de sodium ou un alginate de potassium.

18. Procédé selon l'une quelconque des revendications 1 à 16, **caractérisé en ce que** ledit composant (a) est de l'alginate.

19. Procédé selon l'une quelconque des revendications 1 à 16, **caractérisé en ce que** ledit composant (a) est de l'alginate de potassium.

20. Procédé selon l'une quelconque des revendications 1 à 16, **caractérisé en ce que** ledit composant (a) est de l'alginate de sodium.

21. Procédé selon l'une quelconque des revendications 1 à 20, **caractérisé en ce que** ledit composant acide capable de fournir une libération contrôlée de protons est la glucono-delta lactone.

22. Procédé selon l'une quelconque des revendications 1 à 21, **caractérisé en ce que** le rapport molaire du composant (a) par rapport au composant (b) est inférieure à 30 :1.

23. Procédé selon l'une quelconque des revendications 1 à 21, **caractérisé en ce que** le rapport molaire du composant (a) par rapport au composant (b) est inférieure à 22 :1.

24. Procédé selon l'une quelconque des revendications 1 à 21, **caractérisé en ce que** le rapport molaire du composant (a) par rapport au composant (b) est inférieure à 6 :1.

25. Procédé selon l'une quelconque des revendications 1 à 24, **caractérisé en ce que** le rapport molaire des fractions acides du composant (c) par rapport au nombre de moles du composant (a) est supérieur à 1:1.

26. Procédé selon l'une quelconque des revendications 1 à 24, **caractérisé en ce que** le rapport molaire des fractions acides du composant (c) par rapport au nombre de moles du composant (a) est supérieur à 10:1.

27. Procédé selon l'une quelconque des revendications 1 à 24, **caractérisé en ce que** le rapport molaire des fractions acides du composant (c) par rapport au nombre de moles du composant (a) est supérieur à 100:1.

28. Procédé selon l'une quelconque des revendications 1 à 27, **caractérisé en ce que** la quantité du composant (a) administrée par repas est comprise entre 0,5 à 5,0 grammes.

29. Procédé selon l'une quelconque des revendications 1 à 27, **caractérisé en ce que** la quantité du composant (a) administrée par repas est comprise entre 0,75 à 3,0 grammes.

30. Composition comprenant :
(a) un alginate ou un sel d'alginate apte à la gélation ionotropique,
(b) au moins une source de cations multivalents sélectionnés parmi les sels de calcium, de fer, de strontium, de baryum, de chrome, de manganèse, de nickel, de cobalt, de molybdène, de cuivre, d'aluminium, et de zinc, capable de se solubiliser sous pH acide,
(c) une lactone d'acide carboxylique capable de fournir une libération contrôlée de protons en quantité suffisante pour solubiliser lesdits cations multivalents, et
(d) un composant gazogène,
ladite composition étant apte à s'hydrater dans un milieu aqueux et ensuite à former un gel dans l'estomac lorsque ingérée qui résiste aux forces péristaltiques et qui reste dans l'estomac pendant une période de temps prolongée.

31. Composition comprenant :
(a) un alginate ou un sel d'alginate apte à la gélation ionotropique,
(b) au moins une source de cations multivalents sélectionnés parmi les sels de calcium, de fer, de strontium, de baryum, de chrome, de manganèse, de nickel, de cobalt, de molybdène, de cuivre, d'aluminium, et de zinc, capable de se solubiliser sous pH acide, et
(c) une lactone d'acide carboxylique capable de fournir une libération contrôlée de protons en quantité suffisante pour solubiliser lesdits cations multivalents servant au traitement de l'obésité.

32. Composition selon la revendication 31, comprenant en outre un composant gazogène.

33. Composition selon l'une quelconque des revendications 30 à 32, comprenant en outre un agent dispersant.

34. Composition selon la revendication 33, **caractérisée en ce que** ledit agent dispersant est un solide.

35. Composition selon la revendication 34, **caractérisée en ce que** ledit solide est un sucre cristallin et sélectionné parmi au moins le sorbitol, le fructose, le polydextrose, le sucrose, le maltose, le maltitol, le xylitol ou le mannitol.

36. Composition selon la revendication 35, **caractérisée en ce que** ledit sucre cristallin est du fructose.

37. Composition selon la revendication 34, **caractérisée en ce que** ledit solide est une protéine.

38. Composition selon la revendication 33, **caractérisée en ce que** ledit agent dispersant est un liquide.

39. Composition selon l'une quelconque des revendications 30 et 32 à 37, **caractérisée en ce que** ledit composant gazogène est sélectionné parmi le bicarbonate de sodium, le bicarbonate de potassium, le bicarbonate d'ammonium et le carbonate de calcium.

40. Composition selon la revendication 39, **caractérisée en ce que** ledit composant gazogène est du bicarbonate de sodium.

41. Composition selon la revendication 39, **caractérisée en ce que** ledit composant gazogène est du bicarbonate de potassium.

42. Composition selon l'une quelconque des revendications 30 à 41, comprenant en outre jusqu'à 0,5°/ par poids d'un composant acide capable de fournir une libération immédiate de protons.

43. Composition selon la revendication 42, **caractérisée en ce que** ledit acide capable de fournir une libération immédiate de protons est de l'acide malique.

44. Composition selon l'une quelconque des revendications 30 à 43, comprenant en outre au moins un ingrédient actif sélectionné parmi: les actifs pharmaceutiques, les nutriments, les nutriceutiques, les vitamines, les minerais, les protéines, les probiotiques et les prébiotiques.

45. Composition selon l'une quelconque des revendications 30 à 44, **caractérisée en ce que** ledit composant (a) est un alginate.

46. Composition selon l'une quelconque des revendications 30 à 44, **caractérisée en ce que** ledit composant (a) est un alginate de sodium.

47. Composition selon l'une quelconque des revendications 30 à 44, **caractérisée en ce que** ledit composant (a) est un alginate de potassium.

48. Composition selon l'une quelconque des revendications 30 à 44, **caractérisée en ce que** ledit composant (a) est un mélange d'alginate de sodium et de pectate de sodium faiblement méthoxylé.

49. Composition selon l'une quelconque des revendications 30 à 44, **caractérisée en ce que** ledit composant (a) est un mélange d'alginate de potassium et de pectate de sodium faiblement méthoxylé.

50. Composition selon l'une quelconque des revendications 30 à 44, **caractérisée en ce que** ledit composant (a) est de l'alginate mélangé avec de l'iota-carraghénane.

51. Composition selon l'une quelconque des revendications 30-50 **caractérisée en ce que** ledit composant acide capable de fournir une libération contrôlée de protons est la glucono-delta lactone.

52. Composition selon l'une quelconque des revendications 30 à 51, **caractérisée en ce que** le rapport molaire du composant (a) par rapport au composant (b) est inférieure à 30 :1.

53. Composition selon l'une quelconque des revendications 30 à 51, **caractérisée en ce que** le rapport molaire du composant (a) par rapport au composant (b) est inférieure à 22 :1.

54. Composition selon l'une quelconque des revendications 30 à 51, **caractérisée en ce que** le rapport molaire du composant (a) par rapport au composant (b) est inférieure à 6 :1.

55. Composition selon l'une quelconque des revendications 30 à 54, **caractérisée en ce que** le rapport molaire des fractions acides du composant (c) par rapport au nombre de moles du composant (a) est supérieur à 1:1.

56. Composition selon l'une quelconque des revendications 30 à 54, **caractérisée en ce que** le rapport molaire des fractions acides du composant (c) par rapport au nombre de moles du composant (a) est supérieur à 10:1.

57. Composition selon l'une quelconque des revendications 30 à 54, **caractérisée en ce que** le rapport molaire des fractions acides du composant (c) par rapport au nombre de moles du composant (a) est supérieur à 100:1.
